(19) Europäisches Patentamt
European Patent Office
Office européen des brevets

(11) **EP 4 730 349 A1**

## (12) EUROPEAN PATENT APPLICATION

(43) Date of publication:
**22.04.2026 Bulletin 2026/17**

(21) Application number: **24206753.6**

(22) Date of filing: **15.10.2024**

(51) International Patent Classification (IPC):
**G16H 20/17** (2018.01)     **G16H 50/20** (2018.01)

(52) Cooperative Patent Classification (CPC):
**G16H 50/20; G16H 20/17;** G16H 20/60

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Universität Bern**
**3012 Bern (CH)**

(72) Inventors:
- **MOUGIAKAKOU, Stavroula-Georgia**
  **3045 Meikirch (CH)**
- **PANAGIOTOU, Maria**
  **3008 Bern (CH)**
- **BRIGATO, Lorenzo**
  **3008 Bern (CH)**

(74) Representative: **Perreaud, Jérémie**
**André Roland SA**
**IP Attorneys & Services**
**Avenue Collonges 21**
**1004 Lausanne (CH)**

## (54) ADAPTIVE DRUG MANAGEMENT SYSTEM

(57) The present document discloses an adaptive drug management system for personalized diabetes treatment to a person, comprising at least one of a data collection module configured to store person's blood glucose measurement data and insulin dosage information used to treat the person, a bolus calculator configured to determine a bolus insulin, and a learning module comprising a first agent configured to fine-tune a physiological state (PS) value of the person over time. The system may be configured to retrieve, from the data collection module, the blood glucose measurements taken over a predetermined period, retrieve, from the data collection module, the insulin dosage information used over the predetermined period, update, by the learning module using a reinforcement learning algorithm, the PS value based on the retrieved data, and/or use, by the bolus calculator, the updated PS value to determine the appropriate bolus insulin for the person.

FIG. 1

**Description**

**Technical Field**

The present invention relates to systems and methods for adaptive drug management in the field of diabetes care. More specifically, it involves the use of advanced algorithms, such as reinforcement learning, to optimize and personalize drug dosing for people with diabetes (PwD). These systems may be applied across various devices and platforms, including continuous glucose monitors (CGM), insulin pumps, insulin pens, long-acting insulin formulations, and blood glucose monitoring devices such as self-monitoring blood glucose (SMBG) systems or other types of blood glucose monitors (BGM). The invention aims to improve treatment accuracy and glycemic control regardless of the person's type of diabetes.

**Background**

Diabetes mellitus is a growing global health challenge, increasingly impacting populations worldwide. Achieving optimal glucose control is essential to reduce the risk of complications and improve the quality of life for individuals with diabetes. For those with type 1 diabetes (T1D) and many with insulin-treated type 2 diabetes (T2D), personalized insulin treatment is the core glucose-lowering therapy.

Treatment decision support solutions have been developed to assist PwD in optimizing their daily insulin dosing. However, the effectiveness of these systems is limited, and many do not achieve recommended targets. Various artificial intelligence-based (AI) approaches have been proposed for personalized adjustment of insulin administration for people with T1D, often within closed or hybrid system settings.

Approaches such as standard bolus calculators and closed/hybrid loop systems, although effective in some contexts, often require continuous monitoring and frequent manual adjustments by users. These systems do not always account for individual physiological variations in real-time, limiting their overall effectiveness.

Currently, the most advanced algorithms heavily rely on continuous glucose monitor (CGM) data, which provides a large amount of minute-by-minute data. This approach allows a microscopic focus on blood glucose fluctuations, offering very precise adjustments but requiring ongoing management and interpretation of data. This can lead to information overload for PwD and clinicians and does not always adequately address the broader daily glycemic cycles.

The central challenge is to develop and validate a diabetes management system that is both effective and simplified, aligning insulin dose optimization with people's daily glycemic cycles while minimizing the complexity of daily management. Current state-of-the-art systems, despite their technological advancements, do not address this issue. They primarily focus on minute adjustments based on CGM data, leading to information overload and frequent manual adjustments. These approaches, although successful, do not sufficiently account for individual physiological variations and overall daily glycemic cycles, thereby limiting their effectiveness and practicality for individuals with diabetes.

Unlike traditional systems that focus on immediate fluctuations in blood glucose levels, the present document introduces the possibility of taking long-term variations into account by exploiting a reduced number of data points over extended periods. This approach simplifies the data collection process while providing information on the person's glycemic behavior. By analyzing blood glucose trends at different times of the day, the system can better adapt to changes in the person's condition, improving long-term treatment results and reducing the need for constant manual adjustments. In addition, the system may comprise a self-learning algorithm to improve treatment day by day. These features ensure that the system evolves with the person's needs over time, making it more robust and easier to manage in the long term.

**Summary**

Unlike traditional approaches that focus on minute-by-minute adjustments, the present document discloses a solution which adopts a macroscopic perspective aligned with PwD daily glycemic cycles and lifestyle. The solution may offer a personalized decision support approach for adjusting basal and bolus insulin for individuals with T1D and T2D. This approach reduces the complexity of daily management by focusing on overall trends rather than instantaneous fluctuations. Furthermore, the solution enables the system to continuously learn and adapt based on collected data, improving the accuracy of the recommended insulin doses. The system may comprise several specialized agents, each optimizing treatment parameters specific to a period.

According to a first aspect, the present document discloses an adaptive drug management system for personalized diabetes treatment to a PwD, comprising:

- a data collection module configured to store person's blood glucose measurement data and insulin dosage information used to treat the person,

- a bolus calculator configured to determine a bolus insulin, and

- a learning module comprising a first agent configured to fine-tune a physiological state (PS) value of the individual with diabetes over time.

The system may be configured to:

- retrieve, from the data collection module, the blood glucose measurements taken over a predetermined period,

- retrieve, from the data collection module, the insulin dosage information used over the predetermined period,

- update, by the learning module using a reinforcement learning algorithm, the physiological state (PS) value based on the retrieved data, and

- use, by the bolus calculator, the updated physiological state (PS) value to determine the appropriate bolus insulin for the individual with diabetes.

The reinforcement learning algorithm may comprise an actor-critic algorithm. For example, the learning module may comprise an actor component responsible for improving the control policy and a critic component responsible for evaluating the control policy and providing a temporal difference error for policy optimization.

The learning module may be configured to:

- update the PS value at mealtimes, or at each meal, or upon announcement of a meal by the person or at the person's request, or

- update the PS value using at least two distinct PS agents specific to different times of the day.

The learning module may comprise a second agent configured to update the insulin-to-carbohydrate ratio (ICR) value, and the system may be configured to use, by the bolus calculator, the ICR value to determine the appropriate bolus insulin for the person's meal.

The bolus calculator may consider the person's carbohydrate intake information in determining the bolus insulin. The bolus calculator may provide insulin dosage recommendations for both meals and correction boluses.

The system may be configured to be compatible with multiple types of insulin delivery devices, including insulin pumps and pens.

The learning module may comprise a third agent configured to update a basal insulin dosage of the person with diabetes.

The data collection module may be configured to receive data from a CGM, a SMBG, and/or other BGM type.

The predetermined period may be greater than 12 hours or 24 hours. The data collection module may collect data over a period greater than 24 hours.

The system may comprise an initialization module configured to process the collected data to initialize a policy parameter vector and select algorithm hyper-parameters based on the collected data. The initialization module may filter collected measurement data using a CGM to initialize hyper-parameters. The initialization module may use transfer entropy to initialize the policy parameter vector.

The bolus calculator may take into account the insulin-on-board (IOB) when determining the appropriate bolus insulin for the person with diabetes. The bolus calculator may consider a correction factor to adjust insulin dosage based on the person's current blood glucose level.

The control policy may consist of a linear deterministic policy and a supervisory policy. The final policy may be the weighted sum of the linear deterministic policy (LP) and supervisory policy (SP) wherein the weight is to balance the contribution of LP and SP policy to the final policy of the ICR agents. The linear deterministic policy relates the hypo- and hyperglycemic status to the needed percentage change in the basal insulin, ICR, or PS for the next update. The supervisory policy may provide conservative guidance to ICR agents by acting as a safety measure.

According to a second aspect, the present document discloses an adaptive insulin management system for personalized diabetes treatment to a person with diabetes, the system comprising:

- a data collection module configured to store person's blood glucose measurement data and insulin dosage information used to treat the individual with diabetes,

- a bolus calculator configured to determine a bolus insulin, and

- a learning module comprising a first agent configured to update a PS value of the person and a second agent configured to update an ICR value.

The system may be configured to:

- retrieve, from the data collection module, the blood glucose measurements taken over a 24-hour period,

- retrieve, from the data collection module, the insulin dosage information used over the 24-hour period,

- update, by the learning module using a reinforcement learning algorithm, the PS value and the ICR value based on the retrieved data, and

- use, by the bolus calculator, the updated PS value and the updated ICR value to determine the appropriate bolus insulin for the person with diabetes.

According to one embodiment, the system is designed to operate in cycles (in relation to the person's own cycle, for example, a daily cycle or longer, depending on the type of insulin used), where each time period consists of several successive events. Instead of relying on precise time measurements, the system uses a sequence of events to determine which data to use (e.g., postprandial measurement from the previous day's dinner). Time is interpreted as a relative variable, separating events and potentially ordering them in the sequence. For example, before updating an agent (such as basal insulin calculation, ICR, PS, or CF), the system may require a new blood glucose measurement if the last one is considered too old (e.g., 60 minutes) before the triggering event. The system can then adjust treatment parameters by considering temporal proximity and event sequence rather than strictly adhering to chronological measures, which might be irrelevant, especially when an event is skipped (e.g., skipping lunch).

This description introduces a framework where system decisions are dynamic and based on successive events, thereby improving personalized treatment according to the patient's actions.

Additionally, while this document primarily focuses on the use of insulin as a treatment, other medications may also be employed to enhance the quality of life for patients. These drugs could be integrated into the system to help regulate blood glucose levels in individuals with diabetes.

**Brief Description of Drawings**

The present disclosure will be better understood at the light of the following detailed description which contains non-limiting examples illustrated by the following figures.

Figs.1, 2, 3, and 4 show possible embodiments.
Fig. 5 illustrates how the data can be used to update the basal insulin.
Fig. 6 and 7 illustrate how the data can be used to update the ICR, PS and/or CF values.
Fig. 8 shows an algorithmic description of the update and predict functions.
Fig. 9 shows an example of workflow before the on-line learning.
Fig. 10 shows an algorithmic description of the ABBA Pipeline.

**List of elements**

1      System

2      Delivery device

3      BG device

4      Controller

5      Patient

6      Data collecting module

| | |
|---|---|
| 7 | Bolus calculator |
| 8 | Learning module |
| 9 | Carbohydrate |
| 11 | First agent |
| 12 | Second agent |
| 13 | Third agent |
| 100 | Previous day |
| 101 | Day |
| 102 | Subsequent day |
| 103 | Time period |
| 104 | Time window |
| 105 | Sleeping |
| 106 | First data |
| 107 | Second data |
| 108 | Other measurements |
| 201 | Day one |
| 202 | Day two |
| 203 | Day three |
| 210 | Breakfast |
| 211 | Lunch |
| 212 | Dinner |
| 213 | Snack |
| 214 | BG measurements |
| 215 | First set of data |
| 216 | Second set of data |
| 217 | Third set of data |
| 218 | Fourth set of data |

**Detailed description**

The following detailed description includes references to the accompanying drawings, which form a part of the detailed description. The drawings show, by way of illustration, specific embodiments in which the disclosure may be practiced. These embodiments, which are also referred to herein as "examples," are described in enough detail to enable those skilled in the art to practice the disclosure. The embodiments may be combined, other embodiments may be utilized, or structural, logical and electrical changes may be made without departing from the scope of the present disclosure. The following detailed description is, therefore, not to be taken in a limiting sense, and the scope of the present disclosure is defined by the appended claims and their equivalents.

All scientific and technical terms used herein have meanings commonly used in the art unless otherwise specified. The definitions provided herein are to facilitate understanding of certain terms used frequently herein and are not meant to limit the scope of the present disclosure.

As used in this specification and the claims, the singular forms "a", "an", and "the" encompass embodiments having plural referents, unless the content clearly dictates otherwise.

As used in this specification and the claims, any direction referred to herein, such as "top", "bottom", "left", "right", "upper", "lower", and other directions or orientations are described herein for clarity in reference to the figures and are not intended to be limiting of an actual device or system unless the content clearly dictates otherwise. Devices and systems described herein may be used in several directions and orientations.

As used in this specification and the claims, "have", "having", "include", "including", "comprise", "comprising" or the like are used in their open-ended sense, and generally mean "including, but not limited to".

As used in this specification and the claims, the term "or" is generally employed in its sense including "and/or" unless the content clearly dictates otherwise.

As used in this specification and the claims, "at least one of A, B, and C", "at least one of A, B or C", "selected from the group consisting of A, B, C, and combinations thereof" or the like are used in their open ended sense including " only A, or only B, or only C, or any combination of A, B and C" unless the content clearly dictates otherwise.

As used in this specification and the claims, a "meal" is generally employed in its sense including any occasion where food/drink is consumed/taken. It can vary in size and content, ranging from snacks to larger, structured food/drink intakes like breakfast, lunch, or dinner. A meal typically provides nourishment at different times of the day.

As used in this specification and the claims, a "main meal" is generally employed in its sense including one of the principal eating events during the day, typically consisting of a more substantial portion of food compared to snacks. Examples include breakfast, lunch, or dinner. Main meals generally contain more diverse and nutrient-dense components, playing a key role in a person's daily caloric intake.

As used in this specification and the claims, the terms "patient", "individual with diabetes", "person with diabetes", "PwD", and more broadly "person", refer to any individual living with diabetes, without carrying any negative or stigmatizing connotation. The term "patient" is intended to signify a person receiving medical care, but is used in a respectful and person-centered context. Additionally, "patient" or "person" may be used for brevity and ease of description in the specification or claims.

System description:

Diabetes is a chronic metabolic disorder caused by either the failure of the body to produce insulin - T1D, or the inability of the body to respond adequately to circulating insulin - T2D. Diabetes management is complex and aims at reducing the risk of both short- (e.g., hypo- and hyperglycemia) and long-term complications (e.g., retinopathy, coronary heart disease, nephropathy) by keeping the blood glucose (BG) concentration in the near normal range (glucose control). Self-monitoring of blood glucose (SMBG), continuous glucose monitoring (CGM) and flash glucose monitoring (FGM) are commonly used by PwD to measure glucose concentrations, while for people with T1D and people with T2D under insulin therapy, insulin delivery devices are needed and specifically pens for the case of multiple daily injections (MDI) or pumps for continuous subcutaneous insulin infusion therapy. In any case, PwD under insulin therapy need to visit their physicians every three to four months to check and review the glucose records and perhaps to adjust a) basal insulin, which maintains glucose concentration at consistent levels during periods of fasting, and b) the insulin-to-carbohydrate ratio (ICR), which is essential for calculating the insulin needed to compensate for the effect of the meal (bolus dose).

The need for better BG control is crucial for all PwD, including underrepresented populations. The system disclosed by the present document aims to provide a clinically validated, effective, trustworthy, and cost-efficient AI-based solution for personalized diabetes management (T1D and T2D) that is independent of specific glucose monitoring or delivery devices. The system may be configured to offer daily insulin treatment adjustments to ensure optimal glucose control using (for example) a self-learning approach based on reinforcement learning (RL). This approach is data-driven, may operate in real-time, and is of low computational cost. It enables daily adjustment of insulin and throughout the day, whenever a meal event occurs, on the basis of person's glucose levels fluctuations. Unlike other AI systems that rely on pre-trained models with fixed weights, this system may use on-line learning, allowing the algorithm to dynamically adjust with each new blood glucose measurement, continually refining the suggested values.

The system disclosed in the present document is configured to meet the needs of all PwD, i.e., both people with T1D and people with T2D under insulin-treatment. Thus, the system is designed to accommodate different devices most suitable for the type of diabetes the person has.

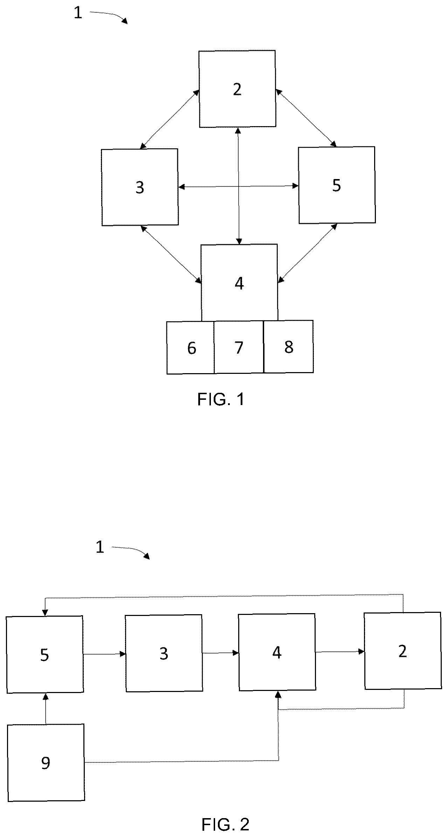

According to various embodiments as shown by Fig. 1, the system (1) may comprise at least one of a delivery device (2), a blood glucose measurement device (3), and a controller (4). The delivery device (2) is configured to administer a drug solution to a patient (5) according to a specified dosage for treatment. The delivery device (2) may include pens for MDI or pumps for continuous subcutaneous insulin infusion therapy. The blood glucose measurement device (3) is configured to measure the person's blood glucose levels and may include SMBG devices, CGM systems, or FGM systems.

The controller may be a part of the delivery device (2) and/or the BG measurement device (3). The controller may be smart phone or a PDA or another distinct device. The controller (4) may be designed to receive data from the BG device (3) and use this data to determine the drug dosage to be administered by the delivery device (2). The controller (4) may comprise advanced algorithms to calculate at least one of an ICR value, a PS value, a bolus insulin, a basal insulin, and a CF, ensuring personalized and precise drug treatment. The system (1) aims to provide comprehensive diabetes management by integrating these components to optimize glucose control and reduce the risk of complications.

The controller (4) may comprise a data processor and several key components designed to enhance diabetes management and optimize treatment outcomes. These components may comprise at least one of:

- Data collection module (6): This module may be configured to store the person's blood glucose measurement data and/or insulin dosage information. It ensures that relevant data, including, but not limited to, historical blood glucose levels, insulin doses, carbohydrate intake, and other pertinent health metrics, are accurately recorded and securely stored.

- Bolus calculator (7): The bolus calculator may be configured to determine the appropriate bolus insulin dose needed to manage blood glucose variation. It may take into account various factors such as one of the ICR, current blood glucose level, target blood glucose level, CF, and IOB.

- Learning module (8): The learning module may be configured to fine-tune parameters over time. The learning module may be configured to use advanced algorithms, such as machine learning and reinforcement learning. This module may analyze the collected data to adjust at least one of the ICR, PS, and basal insulin values. By learning from the person's unique response patterns, the learning module ensures personalized and adaptive insulin therapy, improving overall diabetes management. The learning module may comprise the ABBA (Adaptive Basal-Bolus Advisor) algorithm.

In one embodiment, the learning module may be designed for individuals with T1D and/or individuals with T2D. The learning module may be configured for an individual using either CGM, FGM, or SMBG for glucose monitoring. The learning module may be configured for an individual using either pumps or pens for insulin delivery.

In one embodiment, the controller is a portable device operated by the person with diabetes.

In one embodiment, the data collection module (6), the bolus calculator (7), and the learning module (8) may be arranged in the controller. In this case, all computations are performed by the processor of the controller.

In another embodiment, the data collection module (6) and/or the bolus calculator (7) may be arranged in the controller while the learning module (8) may be arranged in a separate device, for example a remote server. This configuration can be advantageous when the computation requires a large amount of resources. In this case, the controller may include a first communication device, and the remote server may include a second communication device, allowing them to communicate with each other.

For example, the first communication device may be configured to send the data required for the computation to the remote server containing the learning module, and the second communication device may be configured to receive the data from the controller and send back the result of the computation. The first communication device may be also configured to receive the result of the computation.

In another embodiment, the bolus calculator (7) and the learning module (8) may both be arranged in a separate device, for example a remote server, as disclosed above. In this case, the data collection module (6) may be configured to gather all data required for the bolus calculator and the learning module and the controller send these data to the remote controller via the first communication device.

In one embodiment shown in Fig. 2, the system (1) is configured to adapt the insulin management system for personalized diabetes treatment to the individual with diabetes (5). The BG measurement device (3) takes measurements of the person's blood glucose. These measurements may be stored in the data collection module of the controller (4). The data collection module may further store at least one of the dosage/delivery information used by the delivery device (2) to administer the insulin (basal insulin or bolus insulin) and the carbohydrate (9) intake by the person. The controller (4) is configured to gather these data to fine-tune at least one agent of the learning module (also referred to as ABBA).

According to an embodiment, the system may be configured to personalize at least one of the bolus insulin and the basal insulin. The past and current BG measurements and carbohydrates (CHO) intake information may be needed to compute the bolus insulin. For the calculation, it uses the bolus calculator ($BC_{ps}$) formula and adapts the ICR and the current PS of the subject as follows:

$$BC_{PS} = \left( \frac{CHO}{ICR} + \frac{G_c - G_T}{CF} \right) \cdot PS - IOB$$

The PS is subject to large intra- and inter-individual variation, and since there is no research supporting quantification of the proportion, the patient is left to a trial-and-error approach. Differently, the system may be configured to adapt the PS factor based on the glycaemia profile of the subject. Finally, if insulin from previously administered boluses is still active, IOB may be subtracted.

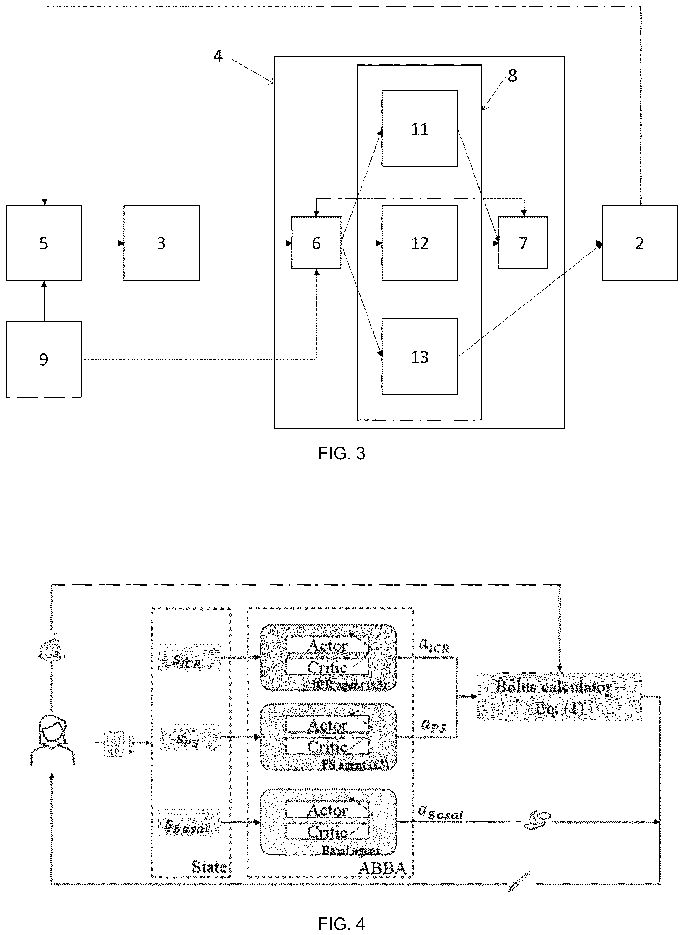

In one embodiment, the learning module may comprise only one agent or two or more agents. As shown in Fig. 3, the learning module (8) of the controller (4) may comprise at least one of the following:

- a first agent (11) may be configured to fine-tune the PS value of the person over time. This agent may be configured to adapt the final bolus insulin dose in response to changes in the glucose profile.

- a second agent (12) may be configured to fine-tune the ICR value of the person over time, and

- a third agent (13) may be configured to fine-tune the basal insulin value.

The first and second agents may also be referred to as bolus agents, while the third agent may be referred to as the basal agent.

In one possible embodiment, the learning module may further comprise a fourth agent configured to fine-tune a correction factor of the person over time.

The data collection module (6) may be configured to gather data and send it to one or more agents. The bolus calculator module (7) is configured to receive the data sent by the first and/or second agents (e.g., PS and ICR) to compute the bolus insulin dose. The bolus calculator may further take into account a correction factor and/or IOB value.

Fig. 4 shows a diagram of one embodiment using an actor-critic for each agent. The learning module may comprise at least 2 (e.g., 3) distinct first agents and/or at least 2 (e.g., 3) distinct second agents. Each agent may be specific for time of the day (e.g., Breakfast, Lunch, Dinner, Snack, ...).

The system may be configured to update at least one of the ICR and the PS value for example for, before or after the meal. The algorithm may be configured to update the basal insulin before the injection of the basal insulin by performing a real-time update. In the case of ICR and PS, the system may comprise three different agents for the ICR and three other agents for the PS: one for breakfast, one for lunch, and one for dinner.

In one embodiment, the system may comprise actor-critic (AC) algorithm, a type of RL algorithms, which may be characterized by two complementary agent parts: the critic, responsible for evaluating the control policy, and the actor, responsible for improving it. The first agent may comprise an AC algorithm configured to fine-tune the PS value of the person over time, the second agent may comprise an AC algorithm configured to fine-tune the ICR value of the person over time, and the third agent may comprise an AC algorithm configured to fine-tune the basal insulin of the person over time. Nevertheless, in another embodiment, other types of algorithms can be used by the first, second and third agents.

Basal Insulin:

In one embodiment, the system is configured to adjust the basal insulin administration at a predetermined frequency. This frequency may be once per time period which may be comprised between 12 to 24 hours, or extend beyond 24 hours, for example depending on the type of insulin used. The time period is an approximate range and should not be interpreted down to the second, or the minute or the hour. For example, a 24-hour period allows for a flexibility of +/- 1 or 2 hours.

In one embodiment, the system may be configured to adapt the frequency to the type of insulin to be injected (via a pump or a pen). For example, it can support short-acting insulin, daily insulin, or ultra-long-acting insulins such as Tresiba® (insulin degludec) and Icodec (both are trademark owned by Novo Nordisk, where a single injection can cover a period of 42 hours or seven days, respectively). The system may be configured to adjust the time period based on the type of insulin, with the user interface (GUI) allowing the selection of either a time period or insulin type.

The time period may determine when the basal insulin needs to be updated. For instance, if the time period is set to 24 hours, at the end of this 24-hour period, the system may be configured to update the basal insulin either automatically or based on a user-initiated action or event.

The system may comprise a time window during which the user can manually initiate the updating of the basal insulin. For example, the user can define a time window between 9:00 PM and 11:00 PM during which the user can request the basal insulin update. Therefore, if the time period is set to 24 hours, every night, the user can request the basal insulin update. In another case if the time period is set to 24 hours and the time window is comprised between 6:00 AM and 10:00 AM, then every morning the user can request the basal insulin update. This time window may be fixed and repeat daily or according to a defined period.

The system may be configured to open a time window at a determined frequency, during which the person must or can initiate the basal insulin update. In one embodiment, the system may be configured to select the data used in calculations based on the time period and/or recorded events (meals, fasting periods, sleep, time windows, etc.).

Fasting can play a significant role in diabetes management, as during this period, the PwD do not need to compensate for CHO intake (from meals) with rapid-acting insulin (bolus insulin). Additionally, sleep is another key factor, since inactivity means that BG levels are solely influenced by the patient's metabolism. The difference between these two measurements can provide valuable insight into the person's metabolic glucose needs, enabling more precise basal insulin recommendations. The system can be configured to account for these events by retrieving and identifying BG data before and after sleep.

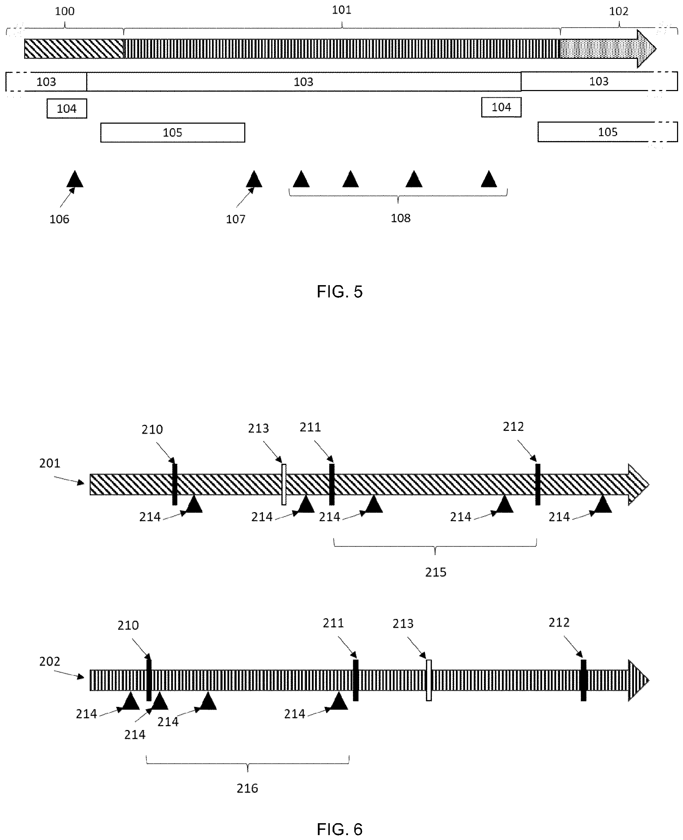

Fig. 5 shows the time with d-1 (100), d (101), and d+1 (102). In this example, the time period (103) is set to 24 hours, the time window (104) is between 8:00 PM and 10:00 PM, and the person sleeps (105) at varying times. The first data (106) may be the BG measurement taken before sleep (this measurement may be defined as the last one before sleep), the second data (107) may be the BG measurement taken after sleep/wake-up (this measurement may be defined as the first one after sleep/wake-up), and the other measurements (108) are performed throughout the time period.

In this example, the data collection module may comprise a plurality of BG measurements taken (for example, at sporadic moments) during the time period. The controller may be configured to identify/define which of these measurements was taken before (e.g., just before) sleep and which one was taken after (e.g., just after) sleep (or wake up).

The process may comprise at least one of the following steps:

- Collect a plurality of blood glucose measurements taken during the time period,

- Identify, from the plurality of BG measurements, a first data (106) which is the BG measurement taken before sleep of the time period (or a last measurement before sleep of the time period),

- Identify, from the plurality of BG measurements, a second data (107) is the BG measurement taken after sleep/wake-up of the time period (or a first measurement before sleep of the time period), and

- Update the basal insulin (which may be used for a next time period), based on the first data (106) and the second data (107), or

- Update the basal insulin (which may be used for the next time period) based on the first data (106) and the second data (107) and other data from the plurality of BG measurements;

- Optionally if the update fails or cannot be computed, retrieve and recommend the last fine-tuned basal insulin value.

The controller may be configured to select which measurements need to be taken into account for the update. The first and second data may be taken into account by the controller in such a way that they are given more weight (higher importance) than the other measurements in the update calculation.

If the time period comprises several sleep periods, the controller may be configured to determine the corresponding measurements for each sleep period, namely the last measurement taken before each sleep phase and the first measurement taken after each sleep phase.

According to one possible embodiment, the system may comprise:

- a data collection module configured to store at least one of a plurality of data related to person's BG measurement and insulin dosage information used to treat the PwD,

- a learning module comprising an agent configured to fine-tune a basal insulin value for a person over time;

The system may be configured to (optionally, following the person's initiation action)

- Retrieve, from the data collection module, a plurality of data relating to BG measurements taken over a predetermined period,

- Optionally, retrieve, from the data collection module, the insulin dosage information used over the predetermined period,

- Identify, from the plurality of data, a first data (106) as a last measurement before sleep of the predetermined period,

- Identify, from the plurality of data, a second data (107) as a first measurement after sleep of the predetermined period, and

- Update, by the learning module, the basal insulin value based on the first data and the second data,

- Optionally if the update fails or cannot be computed, retrieve and recommend the last fine-tuned basal insulin value.

Optionally, the system may be configured to open a time window at a determined frequency, to allow the person to initiate the basal insulin update.

In one embodiment, if the user does not initiate the update during the designated time window, or if the system cannot update the basal insulin due to missing data (for example), the system (since it is designed to improve the recommendation over time) may be configured to suggest the previous recommendation. In other words, if an issue occurs for the computation of the basal insulin dose, the system may be configured to recommend or display the most recent basal insulin dose to the user.

Bolus Insulin:

In one embodiment, the system is configured to calculate or recommend the insulin bolus. An insulin bolus can be administered to regulate the person's BG, particularly when the person eats. Typically, a person consumes at least two main meals and possibly one or more snacks. Main meals generally include breakfast, lunch, and/or dinner, while other smaller meals are considered snacks. Since each meal type impacts the person's metabolism differently, insulin requirements may vary based on the type of main meal consumed. Therefore, the bolus calculator of the system may be configured to take into account the ICR and/or the PS of the PwD.

In order to adapt the bolus insulin to the person with diabetes and to the meal type, the learning module may be configured to finetune at least one of the ICR the PS, and the CF for example at each meal (e.g., at each main meal). In this case, the system may comprise:

- a data collection module configured to store at least one of a plurality of data related to person's BG measurement and insulin dosage information used to treat the person,

- a learning module comprising at least one of a first agent configured to fine-tune a PS value for a person over time, and a second agent configured to fine-tune an ICR value for a person over time.

The system may be configured to allow the person to initiate the update of ICR and/or the PS. In this case, the system may comprise a GUI that enables the person to announce a meal event. For example, the GUI may comprise a screen on which the patient can announce a meal. The GUI may display at least 2 types of meal. The type of meal may comprise at least one of a main meal and a snack. The GUI may display at least two main meals (for example: breakfast, lunch, and/or diner) and optionally a snack. If a main meal is selected by the person, the system may be configured to initiate the update of the ICR and/or PS. If a snack is selected by the person, the system may be configured to use the last fine-tuned ICR and/or PS to calculate the bolus insulin.

In one possible embodiment, the system is configured to update at least one of the ICR, PS, and CF at each or depending on the main meals.

Fig. 6 shows day one (201) and day two (202), the main meals (breakfast (210), lunch (211), and dinner (212)) and snacks (213) consumed by the person, and the BG measurement (214). At lunch (211) of day two (202), the PwD announces the meal (here a lunch (211)). In this example the current meal is lunch on day two.

The system may be configured to:

- Retrieve, from the data collection module, a plurality of data relating to the BG measurements taken over a predetermined period,

- Optionally, retrieve, from the data collection module, the insulin dosage information used over the predetermined period,

- Identify, from the plurality of data, a first set of data (215) comprising BG measurement taken after a determined type of meal,

- Identify, from the plurality of data, a second set of data (216) comprising BG measurement taken before the same determined type of meal.

- Update, by the learning module, the ICR and/or the PS value (and/or CF value) based on the first set of data and the second set of data,

- Optionally if the update fails or cannot be computed, retrieve the last fine-tuned ICR and/or PS value (for example from the last corresponding meal type (here Lunch)).

The determined type of meal may be similar to the type of current meal.

The first set of data may comprise (e.g., only) BG measurement (for example at least one) taken between two consecutive eating events (for example two consecutive main meals). The blood glucose measurement may be the one closest to the meal, for example, the first measurement taken after the meal.

The second set of data may comprise (e.g., only) BG measurement (for example at least one) taken between two consecutive eating events (for example two consecutive main meals). The blood glucose measurement may be the one closest to the meal, for example, the last measurement taken before the meal.

If the person announces a snack, the system may be configured to use the last finetuned ICR, PS, and/or CF value to calculate the insulin bolus or the same value(s) use to calculate the last insulin bolus.

In one embodiment, the system may finetune the values using (e.g., only) data relating to previous meal(s). In this case, the system may be configured to:

- Retrieve, from the data collection module, a plurality of data relating to the BG measurements taken over a predetermined period,

- Optionally, retrieve, from the data collection module, the insulin dosage information used over the predetermined period,

- Identify, from the plurality of data, a first set of data (215) comprising BG measurement taken after a determined type of past meal,

- Identify, from the plurality of data, a second set of data (216) comprising BG measurement taken before the same determined type of past meal.

- Update, by the learning module, the ICR and/or the PS value (and/or CF value) based on the first set of data and the second set of data,

- Optionally if the update fails or cannot be computed, retrieve the last fine-tuned ICR, PS value and/or CF value (for example from the last corresponding meal type (here Lunch)).

The determined type of past meal may be similar to the type of current meal.

For example, to finetune the ICR, PS, and/or the CF used to calculate the insulin bolus for lunch on day two (202), the first set of data may comprise (e.g., only) BG measurements taken between lunch and dinner on a previous day (e.g., day one) while the second set of data may comprise (e.g., only) BG measurements taken between the breakfast and the lunch on a previous day (e.g., day one). The previous day for the first set of data may be the same (or another) as the previous day for the second set of data.

In one embodiment, the system may finetune the values using data relating to previous meal and current meal. In this case, the system may be configured to:

- Retrieve, from the data collection module, a plurality of data relating to the BG measurements taken over a predetermined period,

- Optionally, retrieve, from the data collection module, the insulin dosage information used over the predetermined period,

- Identify, from the plurality of data, a first set of data (215) comprising postprandial BG measurement taken after a previous similar meal, for example: a meal on a previous day, where the meal is of the same type as the current meal (for example, lunch on the previous day when the current meal is lunch),

- Identify, from the plurality of data, a second set of data (216) comprising pre-prandial BG measurement taken before the current meal (for example the current lunch).

- Update, by the learning module, the ICR, PS value, and/or CF value based on the first set of data and the second set of data,

- Optionally if the update fails or cannot be computed, retrieve the last fine-tuned ICR and/or PS value (for example from the last corresponding meal type (here Lunch)).

The first set of data may comprise (e.g., only) the BG measurements taken between two consecutive eating events (for example two consecutive main meals), where the first eating event (of these two consecutive eating events) is a previous similar meal for example, the meal occurred the previous day and which is similar to the current meal.

The second set of data may comprise (e.g., only) the BG measurements taken between two consecutive eating events (for example two consecutive main meals), where the second eating event (of these two consecutive eating events) is the current meal.

For example, the first set of data comprises only the BG measurements taken between lunch and dinner on the previous day (day one) while the second set of data comprises only the BG measurements taken between the breakfast (on the current day) and the current lunch (day two).

In one embodiment, if the person announces a snack, the system may be configured to use the last fine-tuned ICR, PS value, and/or CF value to calculate the insulin bolus. For example, for the snack (213) taken on day two, the bolus calculator will use the ICR and/or PS values that were updated at lunch on day two.

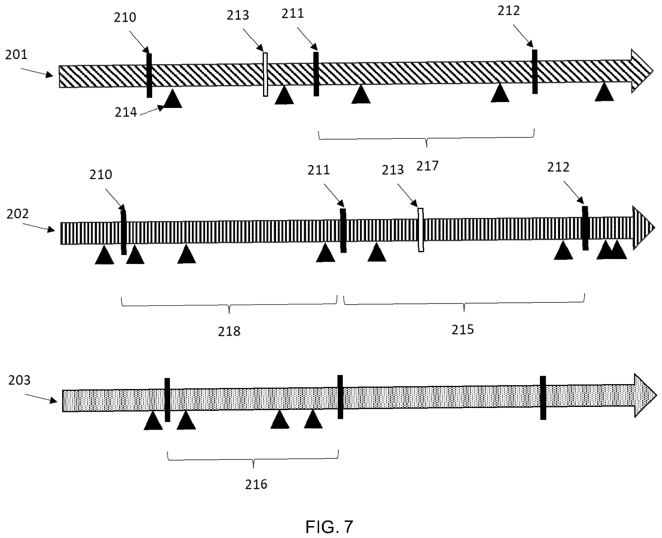

Fig. 7 also shows day three (203). In this example, the current meal is lunch on day three. In this embodiment, the learning module may take into account the BG measurement taken after a similar type of lunch from the previous day.

In one embodiment, the system may be configured to:

- Retrieve, from the data collection module, a plurality of data relating to the BG measurements taken over a predetermined period,

- Optionally, retrieve, from the data collection module, the insulin dosage information used over the predetermined period,

- Identify, from the plurality of data, a first set of data (215) comprising postprandial BG measurements taken after a previous similar meal, for example: a meal on a previous day, where the meal is of the same type as the current meal (for example, lunch on the previous day when the current meal is lunch),

- Identify, from the plurality of data, a second set of data (216) comprising pre-prandial BG measurements taken before the current meal (for example the current lunch).

- Identify, from the plurality of data, a third set of data (217) comprising postprandial BG measurements taken after a meal at least two days ago, where the meal is of the same type as the current meal (for example, lunch from two days ago when the current meal is lunch),

- Identify, from the plurality of data, a fourth set of data (218) comprising pre-prandial BG measurements taken before a meal on the previous day, where the meal is of the same type as the current meal (for example, lunch on the previous day when the current meal is lunch),

- Update, by the learning module, the ICR and/or the PS value based on the first set of data, the second set of data, the third set of data, and the fourth set of data,

- Optionally if the update fails or cannot be computed, retrieve the last fine-tuned ICR and/or PS value (for example from the last corresponding meal type (here Lunch)).

As previously explained, the first set of data may comprise (e.g., only) the BG measurements taken between two consecutive eating events (for example two consecutive main meals), where the first eating event (of these two consecutive eating events) is a previous similar meal for example, the meal occurred the previous day and which is similar to the current meal.

As previously explained, the second set of data may comprise (e.g., only) the BG measurements taken between two consecutive eating events (for example two consecutive main meals), where the second eating event (of these two consecutive eating events) is the current meal.

The third set of data may comprise (e.g., only) the BG measurements taken between two consecutive eating events (for example two consecutive main meals), where the first eating event (of these two consecutive events) is a previous similar meal for example, the meal occurred at least two days ago, and which is similar to the current meal.

The fourth set of data may comprise (e.g., only) the BG measurements taken between two consecutive eating events (for example two consecutive main meals), where the second eating event (of these two consecutive eating events) is a previous similar meal for example, the meal occurred the previous day and which is similar to the current meal.

The difference with the prior art device is fundamental. Here, the system is configured to account for events rather than time itself. Each event is categorized and represents a specific step.

In one embodiment, the data collection module may comprise a first buffer memory to store the first data set and a second buffer memory to store the second data set. The data collection may further comprise a third buffer memory to store the third data set and a fourth buffer memory to store the fourth data set.

In one embodiment, for each main meal, the system may be configured to assign a specific first agent and second agent. In other words, the learning module may include three distinct first agents (one for each main meal) and three distinct second agents (one for each meal). Therefore, the system may be configured to fine-tune each of these agents over time. For example, for lunch, the system may have a lunch first agent and a lunch second agent, both of which are fine-tuned over time by the learning module.

In one embodiment, if the previous similar meal was skipped, the system may be configured to identify data from a similar meal before that.

In one embodiment, if the user does not initiate the update or if the system cannot update the ICR or PS value due to missing data (for example), the system (since it is designed to improve the recommendation over time) may be configured to use the last finetuned ICR or PS value for a similar meal.

System State

The system may be configured to for example daily update over a set of $K = |D|$ days with each day indexed with k ($k \in D = \{1, ..., K\}$). The basal agent may be updated once per day. On each day k, the system may be configured to update the bolus agents $T_k = |\mathcal{M}|$ times with each update $t \in \mathcal{M} = \{t_k, t_{k+1}, ..., T_k\}$. Note that the total number of meal updates $T_k$ may vary across days, e.g., skipping breakfast is allowed. In this work, we fix $T_k = 3$, one for each meal. Finally, BG measurements recorded for an update $t$ are indexed as $\tau \in \mathcal{B} = \{\tau_{t_k}, \tau_{t_k} + 1, ..., S_{t_k}\}$ and for an update k as $\upsilon \in \beta = \{\upsilon_k, \upsilon_k + 1, ..., R_k\}$. The number of measurements per update may vary. For example, on one afternoon, the person might document three readings (one after a meal and two additional ones), whereas on another afternoon, only the post-prandial measurement may be recorded. In this manner, the system may be configured to adapt to a variable number of measurements per step.

Now, define the glucose error $G^\Delta$ with respect to a measurement as:

$$G^\Delta = \begin{cases} G - G_h & if\ G > G_h \\ G - G_l & if\ G < G_l \\ 0 & otherwise \end{cases}$$

with $G_h = 180\ mg/dL$ and $G_l = 70\ mg/dL$ are respectively the hyper- and hypoglycaemia bounds. The building block of the system state and cost is the feature vector $\boldsymbol{F} \in \mathbb{R}^2$. For the basal agent, $\boldsymbol{F}_k$ is computed as:

$$\boldsymbol{F}_k = \left[ \frac{1}{N_h}(\sum_\upsilon G_\upsilon^\Delta + \sum_{t,\tau} G_{t,\tau}^\Delta), -\frac{1}{N_l}(\sum_\upsilon G_\upsilon^\Delta + \sum_{t,\tau} G_{t,\tau}^\Delta) \right]$$

meaning that all recorded measurements over a day, including the bolus recordings, are included. For the bolus agents the features $\boldsymbol{F}_t$ are computed as:

$$\boldsymbol{F}_t = \left[ \frac{1}{N_h}\sum_\tau G_\tau^\Delta, -\frac{1}{N_l}\sum_\tau G_\tau^\Delta \right]$$

For both cases, $N_h$ is the number of samples above the hyperglycaemia and $N_l$ below the hypoglycaemia thresholds. Also, the features may be normalized into the range [0, 1].

Different states may be designed for each group of agents to provide more relevant information and improve prediction ability. The Basal agent implements the state $s_k^{bas} \in \mathbb{R}^4$ as:

$$s_k^{basal} = [\boldsymbol{F}_k, \boldsymbol{b}_k]$$

with $\boldsymbol{b}_k$ containing features quantifying the difference in BG between morning and night measurements. More rigorously, $\boldsymbol{b}_k \in \mathbb{R}^2$ and is defined as:

$$\boldsymbol{b}_k = \begin{pmatrix} \begin{cases} G_{\tau=\tau_{t_k}} - G_{\upsilon=R_{k-1}} & if \ G_{\tau=\tau_{t_k}} > G_h \ and \ G_{\upsilon=R_{k-1}} < G_h \\ 0 & otherwise \end{cases} \\ \begin{cases} G_{\upsilon=R_{k-1}} - G_{\tau=\tau_{t_k}} & if \ G_{\tau=\tau_{t_k}} < G_{l1} \ and \ G_{\upsilon=R_{k-1}} > G_{l1} \\ 0 & otherwise \end{cases} \end{pmatrix}$$

Note that the subscript $\tau = \tau_{tk}$ indicates the first-morning measurement of the current day and $G_{l1}$ = 90 mg/dL. Simply speaking, the components of $\boldsymbol{b}_k$ are different from zero if overnight a hypo- or hyperglycaemic event has occurred. As described above in one embodiment, there may be three ICRs. In this case, the state of the ICR agents with t < $T_k$, i.e., the breakfast and lunch agents, the state is:

$$s_{t<T_k}^{ICR} = \boldsymbol{F}_t$$

In the case of t = $T_k$, meaning the ICR for the dinner meal, the state is modified as follows:

$$s_{t=T_k}^{ICR} = [\boldsymbol{F}_t, \boldsymbol{b}_k]$$

The difference between morning and night measurements may be also concatenated. In summary, $s_{t<T_k}^{ICR} \in \mathbb{R}^2$ and $s_{t=T_k}^{ICR} \in \mathbb{R}^4$. For the PS agents the state is $s_t^{PS} = \boldsymbol{F}_t, s_t^{PS} \in \mathbb{R}^2$.

Design of the Cost Function

ABBA minimizes a modified version of the original cost function. The cost collected after the executed action is:

$$c_{\{t+1,k+1\}} = \boldsymbol{\beta} \cdot \boldsymbol{s}_{\{t+1,k+1\}}[\boldsymbol{0}, \boldsymbol{1}] = \boldsymbol{\beta} \cdot \boldsymbol{F}_{\{t+1,k+1\}}^{\mathrm{T}}$$

where $\beta = [\beta_{hypo}, \beta_{hyper}]$ is a vector containing the weights $\beta_{hypo}$ and $\beta_{hyper}$ used for scaling the hypo- and hyperglycaemia components. Simply speaking, the cost is proportional to the features after the performed action. For people with T1D, the aim may be to minimize the hypoglycemia events. Thus, the weights may be chosen as $\beta_{hyper}$ = 1 and $\beta_{hypo}$ = 10. On the other hand, in the case of people with T2D, the goal may be to reduce the hyperglycemia events. Therefore, the weights may be chosen as $\beta_{hyper}$ = 10 and $\beta_{hypo}$ = 1.

Design of the Critic

The critic agent may be configured to assess the current control policy by estimating the long-term expected cost. The critic component of the system may be updated as detailed below. It provides temporal difference (TD) error to the actor for policy optimization, expressed as:

$$d_{\{t,k\}} = \boldsymbol{c}_{\{t+1,k+1\}} + \gamma \cdot \boldsymbol{V_w}(s_{\{t+1,k+1\}}) - \boldsymbol{V_w}(s_{\{t,k\}})$$

Where $V_w(s_{\{t,k\}})$ is the value function. $V_w(s_{\{t,k\}})$ is parametrised as $V_w(s_{\{t,k\}}) = w \cdot s_{\{t,k\}}$ with w representing the parameter vector w. The critic parameter vector w may be randomly initialized at the beginning and updated according to:

$$w_{\{t+1,k+1\}} = w_{\{t,k\}} + lr_c \cdot d_{\{t,k\}} \cdot z_{\{t,k\}}$$

where $lr_c$ denotes the learning rate and $z_{\{t,k\}}$ is the eligibility vector updated as follows:

$$z_{\{t+1,k+1\}} = \lambda \cdot z_{\{t,k\}} + s_{\{t+1,k+1\}}$$

with $\lambda$ being the eligibility trace decay factor.

Design of the Actor

The actor agent may be configured to optimize the control policy in order to minimize the long-term cost. The control policy (P) may comprise at least one of the linear deterministic policy (*LP*) and the supervisory policy (SP). Specifically, the linear deterministic policy may relate the hypo- and hyperglycemic status to the needed percentage change in the Basal, ICR, or PS for the next update. LP is implemented by a linear layer parameterized by $\theta$:

$$LP_{\{t,k\}} = \theta_{\{t,k\}} \cdot s_{\{t,k\}}$$

The supervisory policy provides conservative guidance to ICR agents by acting as a safety measure. It is described as follows:

$$SP_t = \begin{cases} -\alpha_{SP} \cdot F_{t+1,k-1}[0], & if \; (F_{t+1,k-1}[0] > 0 \; and \; F_{t+1,k-1}[1] = 0) \; or \; F_{t+1,k-1}[0] > F_{t+1,k-1}[1] \\ \alpha_{SP} \cdot F_{t+1,k-1}[1], & if \; (F_{t+1,k-1}[1] > 0 \; and \; F_{t+1,k-1}[0] = 0) \; or \; F_{t+1,k-1}[1] > F_{t+1,k-1}[0] \\ 0, \; i & \quad F_{t+1,k-1}[0] \; = F_{t+1,k-1}[1] = 0 \end{cases}$$

with $\alpha_{SP}$ working as a hyper-parameter to balance the influence of the correction.
Therefore, the final policy may be the weighted sum of the linear deterministic and supervisory policy:

$$P_t = \alpha_{LP} \cdot LP_t + (1 - \alpha_{LP}) \cdot SP_t$$

where $\alpha_{LP}$ may be defined as follows:

$$\alpha_{LP} = \begin{cases} 0.5, & if \; ((F_{t+1,k-1}[0] > 0 \; and \; F_{t+1,k-1}[1] = 0) \; or \; F_{t+1,k-1}[0] > F_{t+1,k-1}[1]) \; or \\ & ((F_{t+1,k-1}[1] > 0 \; and \; F_{t+1,k-1}[0] = 0) \; or \; F_{t+1,k-1}[1] > F_{t+1,k-1}[0]) \\ 0, & if \; F_{t+1,k-1}[0] \; = F_{t+1,k-1}[1] = 0 \\ 1, & otherwise \end{cases}$$

In one embodiment, the role of $\alpha_{LP}$ is to balance the contribution of *LP* and SP policy to the final policy of the ICR agents. When the features are in range, the weighting factor αLP is deliberately set to 0, ensuring that the *LP* policy remains unweighted. Otherwise, equal contributions to both policies are ensured when the features overcome this range. In the case of the basal and PS agents, the final policy is equal to the linear policy.
The actor's policy parameters θ may be updated by following the policy gradient $g_{\{t,k\}}$, which, having a linear policy, simply corresponds to:

$$g_{\{t,k\}} = \frac{d_{\{t,k\}}}{s_{\{t,k\}}}$$

Where $d_{\{t,k\}}$ is the TD error. We employ the Adam optimiser with learning rate $lr_a$ to solve the optimization problem.

Action

For the ICR and PS agents, the final action prediction may be according to the following formula:

$$a_t = a_{t,k-1} + m \cdot P_t \cdot a_{t,k-1}$$

For the basal agent, the action may correspond to:

$$a_k = a_{k-1} + m \cdot P_k \cdot a_{k-1}$$

where m may be experimentally chosen to be 0.5 for T1D and 1.0 for T2D. In this manner, the system may be configured to recommend the basal insulin, ICR, or PS, depending on the specific agent in use. The action space for at is a continuous value in $\left[\frac{\alpha_{init}}{2}, 2 \cdot \alpha_{init}\right]$, where $\alpha_{init}$ represents the initial value from the simulator or physician in real-life settings. To ensure safety, the basal insulin action may be set to at least 25% of the previous day's Total Daily Dose (TDD).

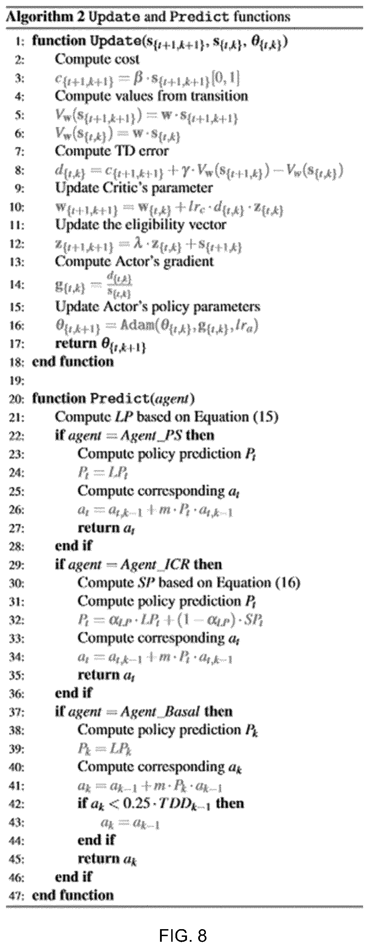

For example, Fig. 8 shows an algorithmic description of the update and predict functions.

Adaptive optimizer

The Adam optimizer may be used during the online learning phase (for example) to update at least one of the actor's policy parameters θ, ensuring efficient and adaptive learning. Unlike other algorithm that relied on a static learning rate, Adam introduces dynamic adjustments to the learning rate by leveraging first-order momentum (the running average of gradients) and second-order momentum (the running average of squared gradients). This allows the algorithm to adapt more effectively to the changing landscape of the optimization problem, making it particularly well-suited for complex environments such as actor-critic learning dynamics where the scale of gradients can vary significantly over time.

Data collection and initialization workflow before on-line learning

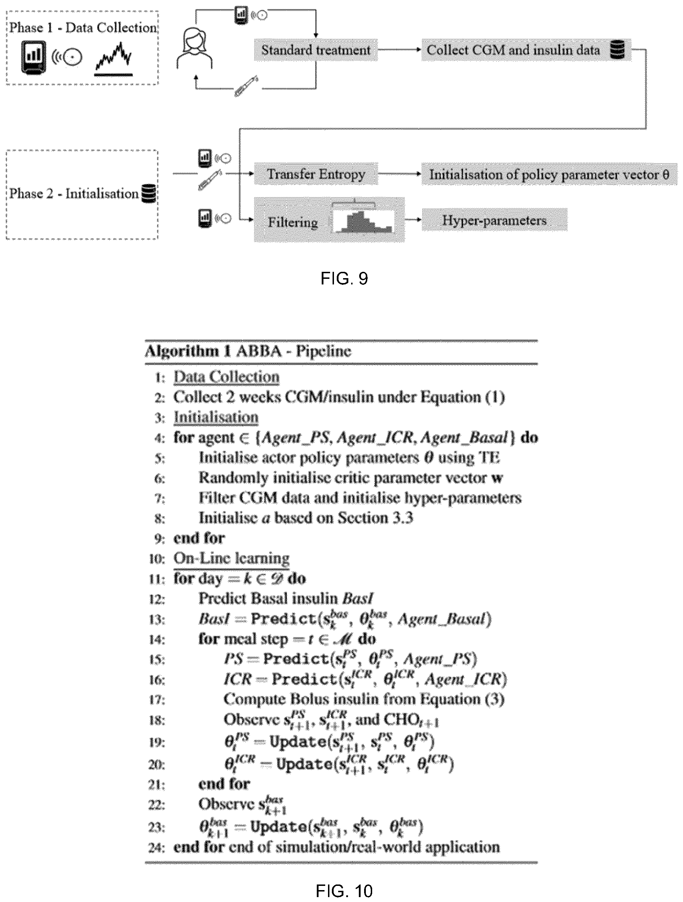

Fig. 9 shows an example of workflow before the on-line learning and Fig. 10 shows an algorithmic description of the ABBA Pipeline.

Phase 1 - Data Collection: The first phase may be the data collection, which may last for two weeks. In this phase, the person with diabetes employs the standard treatment. The person follows the insulin suggestion from the bolus advisor with the values of ICR, CF, and basal insulin provided by the simulator for T1D or by standard equations for T2D. During this phase, the collected data may be at least one of the CGM measurements and insulin dosage information data.

For individuals with diabetes requiring insulin treatment to maintain their blood glucose levels within the safe normoglycemic range, it is recommended to administer slow-acting insulin injections once or twice daily to control fasting blood glucose levels, along with fast-acting or bolus insulin injections before meals to mitigate postprandial glucose spikes. For the data collection period, the bolus insulin may be calculated with the formula of standard BC defined as:

$$BC = \frac{CHO}{ICR} + \frac{G_c - G_T}{CF} - IOB$$

where CHO (g) is the meal carbohydrate intake, ICR (g/U) and CF (mg/dL/U), respectively are the insulin-to-carbohydrates ratio and the correction factor, i.e., two therapy parameters may be tuned by clinicians, Gc (mg/dL) is the mealtime BG level, Gt (mg/dL) is the target BG level, and IOB (U) is the insulin on board, indicating the previously injected insulin that is still acting in the organism.

Phase 2 - Initialization: The second phase may be the initialization, which may be occurred after data collection and before starting on-line learning. The collected data may be used to 1) initialize the policy parameter vector of the system algorithm and 2) select the algorithm hyper-parameters based on the personalized CGM data.

To guarantee safety, the initial values for the ICR, CF, and basal insulin may be specific and appropriate for each person. When implemented in clinical practice, the ICR, CF, and basal insulin of the system are initialized using the individual values of the patient with diabetes as optimized by their physician. To reflect the bias that the healthcare specialist may have when estimating "optimal" values, a uniform uncertainty of $\pm 10\%$ may be randomly applied. The simulator provides the default ICR, CF, and basal insulin for T1D. For T2D, such values may be calculated using the standard equations.

Policy parameters may be variables that define the behavior of the advisor. The initialization of the policy parameter vector θ is based on the transfer entropy (TE) between CGM data and the active insulin. Active insulin (AI) may be estimated as the sum of IOB related to the bolus doses and basal insulin (Basal) infusion collected in the first two weeks:

$$AI = IOB + BasI$$

Hyper-parameters may be settings that guide how ABBA learns, such as the speed of learning or the complexity of the suggested insulin dose. The varied responses to insulin among individuals with diabetes necessitate personalized treatment, as evidenced by discrepancies in the stability of blood glucose profiles. Consequently, those with unstable BG profiles may require more careful management, leading us to adopt more conservative algorithmic updates. Two weeks of BG data from 100 simulated adults diagnosed with T1D may be gathered, and the standard deviation of BG values may be computed. Notably, the CGM profiles display a nearly Gaussian distribution. Individuals with unstable BG patterns are considered those with a z-score > 1 in the standard deviation of the BG profiles. Practically, people with T1D whose BG standard deviation exceeds 57 are classified as unstable. The same approach is repeated for people with T2D, with people with T2D defined as an outlier with a BG standard deviation surpassing 59. Therefore, the AC's initial learning rates are decreased to support those with unstable BG profiles more effectively. Moreover, if the subject in the first two weeks has BG less than 70 mg/dL for more than 21% of the time for T1D and 42% for T2D overnight, they are considered high-risk. Following this approach, the actor learning rate of the dinner ICR may be adjusted.

The hyper-parameters of the ABBA algorithm may be summarized as in Table 1. The hyper-parameters do not differ among the virtual subjects. Symbol → indicates the change of the hyper-parameters when the subject is considered as an outlier, and for hyper-parameters that differ between T1D and T2D, the notation "/" is used to show the specific values for each condition. $\alpha$SP may be set to 0.1. In addition, if the subject is considered as high risk, the initial actor learning rate for ICR of dinner is set to 0.01 in case for patients with normal BG profile in the first two weeks and to 0.001 in case for unstable BG profile in the first two weeks.

Table 1: Hyper-parameters of the ABBA algorithm:

| Hyper-parameter | Symbol | Value |
|---|---|---|
| Discount factor | Y | 0.9 |
| Eligibility trace decay factor | $\lambda$ | 0.5 |
| Critic learning rate for ICR | $lr_c$ | 0.1 → 0.05 |
| Critic learning rate for PS | $lr_c$ | 0.1 → 0.01 |
| Critic learning rate for Basal | $lr_c$ | 0.1 → 0.01 |
| Actor learning rate for ICR | $lr_a$ | 0.1 → 0.01 |
| Actor learning rate for PS | $lr_a$ | 0.1 → 0.01 |
| Actor learning rate for Basal | $lr_a$ | 0.1 → 0.01 |
| Weight of SP | $a_{SP}$ | 0.1 |
| Smoothing factor | m | 0.5/1 |

Phase 3 - On-line Learning: The third phase may consist of the learning process as described above. The system algorithm may be configured to predict the bolus and basal insulin suggestions.

## Experimental example

The objective of this test is to evaluate the performance and efficacy of the system (also called ABBA) algorithm in accurately managing blood glucose levels for individuals with both T1D and T2D. This involves assessing the algorithm's ability to adjust insulin treatment daily based on real, thereby ensuring optimal glucose control. The test aims to validate the extended version of ABBA, tailored specifically for the FDA-accepted individuals with diabetes under MDI therapy and SMBG measurement. We compared ABBA algorithm against the standard treatment.

These tests play a pivotal role in the overall development cycle of ABBA. Firstly, they serve as critical validation steps to ensure the algorithm's effectiveness and safety in real-world scenarios. By conducting rigorous testing under various conditions, including disturbances, uncertainties, and variabilities, developers can identify and address any potential shortcomings or limitations of the algorithm. Moreover, the validation of the extended version of ABBA for individuals with T2D represents a significant advancement in diabetes management. Given the rising prevalence of T2D globally, the inclusion of insulin pens and optimization of insulin dosing parameters tailored to this population is of paramount importance. These tests ensure that the algorithm meets the diverse needs of individuals with diabetes, thereby enhancing its clinical utility and impact on improving patient outcomes. Overall, these tests serve as crucial quality assurance measures, guiding the refinement and optimization of the ABBA algorithm to deliver personalized and effective diabetes management solutions for PwD worldwide.

To test a scenario as close as possible to real-life situations, such as the one that will take place during the clinical trial, we include uncertainties and variabilities in meal announcements, insulin injections, blood glucose measurements, and insulin sensitivity. In this experiment, we required a blood glucose measurement before each meal and one before bedtime per day, measured with virtual SMBG devices. One day of simulation included three main meals per day: breakfast, lunch, dinner, and one snack. Both meal timing and CHO content of the meals were extracted from uniform distributions.

Table 3 reports the minimum and maximum values of the distributions from which the meal timing and CHO amount are drawn.

Table 1: Minimum and Maximum Values of the Possible CHO amount and Time of Consumption for the different meals (Uniform distribution)

| Meal type | CHO amount [g] | Meal timing |
|---|---|---|
| Breakfast | [42 - 98] | [7:00 am - 9:00 am] |
| Lunch | [60 - 140] | [12:30 am - 1:30 pm] |
| Dinner | [54 - 126] | [7:00 pm - 8:00 pm] |
| Snack | [5 - 21] | [10:00 am - 11:00 am] or [3:00 pm - 6:00 pm] or [9:00 pm - 10:30 pm] |

We fix the duration of the experiment to 90 days to match the duration of the expected clinical trial. The algorithm needs 14 additional days at the beginning for the parameters' initialization. The main meal lasts 15 to 30 minutes, and the snack 3 to 8 minutes. The bolus injection and the meal announcement take place 5 to 15 minutes before the meal, and the basal insulin injection from 10:00 pm to 12:00 pm. In addition, we consider that the subjects are likely to under or overestimate the carbohydrate content of meals by 70% and 110%, respectively. Also, we add variability of $\pm$ 10% to the Physicians' ICRs / PSs / Basal. Both variabilities and uncertainties follow uniform distributions. The intraday variability of insulin sensitivity was considered as the "dawn phenomenon" for individuals with T1D. This refers to periodic episodes of hyperglycemia occurring in the early morning hours before and after breakfast. In the present study, SI also dropped every day between 04:00 and 08:00 to 50% of its original value, and SI ramped up or down within around 30 minutes. Finally, we assume that there is no meal intake or bolus injection after the basal injection, since we realistically assumed that the basal injection is taken right before bedtime. In addition, we add a rescue meal controller which is activated when the BG goes below 30 mg/dL and gives to the subject a glucose tablet dose of 20 gram, as suggested from the simulator's documentation. A rescue meal controller in order to avoid extreme hypoglycemic events. During the clinical trial setting this threshold can be adjusted. When the rescue meal controller is activated, ABBA takes into consideration this BG measurement.

The evaluation of the performance of the experimental scenarios was based on the analyses of the BG levels of the virtual adults. Different metrics were implemented to assess the performance. The most widely used parameter is the percentage of time in different blood glucose ranges: percentage time in glucose target range (% in Target) [70-180] mg/dl; percentage time in hypoglycemia (% in Hypo) < 70 mg/dl, and percentage time in hyperglycemia (% in Hyper) > 180 mg/dl. In addition, the mean number of hypo and hyperglycemic events, BG, maximum and minimum BG and the total daily dose (TDD) in units per day of insulin were extracted. Lastly, to evaluate the statistical significance of the resulting metric distribution, we applied the Wilcoxon test with a significance level equal to 1% to the metric % in Target, % in Hypo, and % in Hyper, which showed a non-Gaussian distribution based on the Lilliefors test.

Definition of Success and Failure Criteria to Assess the Algorithm's Effectiveness:

• Success Criteria:

   ◦ Primary Outcome: Percentage time in target range (% in Target) [70-180] mg/dl: The algorithm is considered successful if the virtual adults spend a significant portion of their time within the target glucose range compared to the standard treatment.

   ◦ Improvement of time in target range for the ABBA algorithm over-time.

   ◦ Reduce time spent in hypoglycemia for people with T1D and reduce time spent in hyperglycemia for people with T2D.

• Failure Criteria:

   ◦ Percentage time in hypoglycemia (% in Hypo) < 70 mg/dl: Failure occurs if virtual adults spend an excessive amount of time in hypoglycemic states.

○ Percentage time in hyperglycemia (% in Hyper) > 180 mg/dl: Failure occurs if virtual adults spend an excessive amount of time in hyperglycemic states.

Successful performance is determined by achieving statistically significant improvements in these metrics compared to baseline algorithm. By rigorously evaluating the ABBA algorithm against these success and failure criteria, its effectiveness in managing blood glucose levels can be objectively assessed, providing valuable insights for clinical implementation and further algorithm refinement.

ABBA algorithm is validated in the in-silico on 200 simulated adults (100 with T1D and 100 with T2D) of the FDA-accepted simulator with the Experimental Scenario 1.

Table 4: In-silico results with the full cohort for the first and the last 4 weeks

| | First 4 Weeks | | | Last 4 Weeks | | |
|---|---|---|---|---|---|---|
| | % in Target | % in Hypo | % in Hyper | % in Target | % in Hypo | % in Hyper |
| **Adults - T1D** | | | | | | |
| **BBA** | 70.4 ± 14.1 | 4.5 ± 4.7 | 25.2 ± 12.4 | 71.7 ± 13.9 | 3.5 ± 4.6 | 24.9 ± 12.1 |
| **ABBA** | 72.7 ± 12.5$^t$ | 1.0 ± 1.2$^t$ | 26.3 ± 11.7 | **84.7 ± 11.9**$^t$ | **0.7 ± 0.9**$^t$ | **14.6 ± 11.6**$^t$ |
| **Adults - T2D** | | | | | | |
| **BBA** | 68.4 ± 19.8 | 7.5 ± 12.1 | 24.1 ± 21.7 | 69.5 ± 20.1 | 7.3 ± 12.5 | 23.1 ± 21.9 |
| **ABBA** | 75.9 ± 17.9$^t$ | 1.6 ± 2.4$^t$ | 22.5 ± 18.3$^t$ | **83.7 ± 16.5**$^t$ | **0.6 ± 1.3**$^t$ | **15.7 ± 16.2**$^t$ |
| *Symbol 1 indicates statistical significance (p ≤ 0.01) with respect to the BBA.* | | | | | | |

We compare ABBA algorithm versus the basal-bolus advisor (BBA), which consists of the bolus insulin using the formula above and the basal insulin that is suggested from the simulator. Table 4 presents the results of the in-silico population for both BBA and ABBA algorithms, comparing the performance in the first and the last 4 weeks of the 3-month evaluation. During the first 4 weeks, BBA demonstrated a % in Target of 70.4 ± 14.1 for T1D adults and 68.4 ± 19.8 for T2D adults.

In the last 4 weeks, BBA expectedly maintained a similar performance, with % in Target values of 71.7 ± 13.9 for T1D and 69.5±20.1 for T2D. On the other hand, the ABBA algorithm exhibited better performance, especially for the % in the Hypo category, with values as low as 1.0 ± 1.2 for T1D and an impressive 0.7 ± 0.9 during the last 4 weeks. Notably, there is an absolute improvement of roughly 13% in the glycemic control over time using the ABBA algorithm. This suggests that ABBA may offer enhanced glycemic control, particularly in minimizing hypoglycemic events for T1D, making it a promising candidate for diabetes management. Importantly, the results also highlight the potential of the ABBA algorithm in benefiting individuals with T2D, as evidenced by a substantial improvement in % in Target (83.7 ± 16.5), % in Hypo (0.6 ± 1.3), and % in Hyper (15.7 ± 16.2), during the last 4 weeks.

Table 5: In-silico results with the full cohort in 3-months duration

| | % in Target | % in Hypo | % in Hyper | # Hypo events | # Hyper events | BG mean | BG max | BG min | HbA1c | LBGI | TDD (U) |
|---|---|---|---|---|---|---|---|---|---|---|---|
| **Adults - T1D** | | | | | | | | | | | |
| **BBA** | 70.6±13.8 | 4.4±4.8 | 25.0±12.1 | 35.1±34.0 | 199.4±51.3 | 148±13 | 259±41 | 50±17 | 6.8 ±0.4 | 1.28 ±1.38 | 44.19 |
| **ABBA** | 80.1±10.8† | 0.9±0.9† | 19.0±10.2 † | 10.7±10.7 | 200.3±59.1 | 148±13 | 262±39 | 47±17 | 6.8 ±0.3 | 0.27 ±0.22 | 43.35 |
| **Adults - T2D** | | | | | | | | | | | |
| **BBA** | 68.3±19.7 | 7.9±12.6 | 23.8±21.5 | 52.8±98.2 | 177.0±95.7 | 145±37 | 285±79 | 73±34 | 6.7 ±1.3 | 2.05 ±3.30 | 51.02 |
| **ABBA** | 80.1±16.5† | 1.0±1.3† | 18.9±16.4† | 11.4±18.0 | 190.6±93.9 | 145±22 | 284±73 | 65±28 | 6.7 ±0.8 | 0.34 ±0.36 | 56.47 |
| | Symbol † indicates statistical significance (p ≤ 0.01) with respect to the BBA. | | | | | | | | | | |

Table 5 provides the in-silico result spanning the 3-month duration. In BBA, T1D patients using the current standard treatment exhibited a % in Target of 70.6 ± 13.8, indicating a satisfactory level of glycemic control. However, we also observe notable occurrences of hypoglycemia (% in Hypo: 4.4 ± 4.8) and hyperglycemia (% in Hyper: 25.0 ± 12.1). In contrast, T1D patients under the ABBA algorithm demonstrated an enhanced % in Target (80.1 ± 10.8) with an impressive reduction in hypoglycemic time (% in Hypo: 0.9 ± 0.9). This suggests that the ABBA algorithm offers improved glycemic control, particularly by minimizing the risk of hypoglycemia for T1D. Interestingly, T2D patients following the standard treatment exhibited similar trends, with a % in Target of 68.3 ± 19.7. However, the % in Hypo and Hyper were relatively higher at 7.9 ± 12.6 and 23.8 ± 21.5, respectively. The performance of ABBA for T2D is significantly improved to 80.1 ± 16.5, reducing the hypoglycemic time by approximately 7% and reaching a hyperglycemic time of 18.9 ± 16.4.

**Claims**

1. An adaptive drug management system for personalized diabetes treatment to a person, the system comprising:

   • a data collection module configured to store person's blood glucose measurement data and insulin dosage information used to treat the person,
   • a bolus calculator configured to determine a bolus insulin, and
   • a learning module comprising a first agent configured to fine-tune a physiological state (PS) value of the person over time;

   wherein the system is configured to:

   • retrieve, from the data collection module, the blood glucose measurements taken over a predetermined period,
   • retrieve, from the data collection module, the insulin dosage information used over the predetermined period,
   • update, by the learning module using a reinforcement learning algorithm, the PS value based on the retrieved data, and
   • use, by the bolus calculator, the updated PS value to determine the appropriate bolus insulin for the person.

2. The system of claim 1, wherein the reinforcement learning algorithm comprises an actor-critic algorithm.

3. The system of claim 2, wherein the learning module further comprises:

   • an actor component responsible for improving the control policy;
   • a critic component responsible for evaluating the control policy and providing a temporal difference error for policy optimization.

4. The system of claim 1, wherein the learning module is configured to update the PS value at mealtimes, or at each meal, or upon announcement of a meal by the patient or at the patient's request.

5. The system of claim 1, wherein the learning module is configured to update the PS value using at least two distinct PS agents specific to different times of the day.

6. The system of claim 1, wherein the learning further comprises a second agent configured to update an Insulin to Carbohydrate Ratio (ICR) value and wherein the system is configured to use, by the bolus calculator, the ICR value to determine the appropriate bolus insulin for the person's meal.

7. The system of claim 1, wherein the bolus calculator considers the person's carbohydrate intake information in determining the bolus insulin.

8. The system of claim 1, wherein the bolus calculator provides insulin dosage recommendations for both meals and correction boluses.

9. The system of claim 1, wherein the system is designed to be compatible with multiple types of insulin delivery devices, including insulin pumps and pens.

10. The system of claim 1, wherein the learning module comprises a third agent configured to update a basal insulin dosage of the person.

11. The system of claim 1, wherein the data collection module is configured to receive data from a CGM, a SMBG, and/or other BGM type.

12. The system of claim 1, wherein the predetermined period is greater than 12 hours or 24 hours.

13. The system of claim 1, wherein the data collection module collects data over a period greater than 24 hours.

14. The system of the claim 1 further comprising: an initialization module configured to:

     • process the collected data to initialize a policy parameter vector, and
     • select algorithm hyper-parameters based on the collected data;

15. The system of claim 14, wherein the initialization module filters collected measurement data using a CGM to initialize hyper-parameters.

FIG. 1

FIG. 2

FIG. 3

FIG. 4

FIG. 5

FIG. 6

FIG. 7

**Algorithm 2** Update and Predict functions

1: **function Update**($s_{\{t+1,k+1\}}$, $s_{\{t,k\}}$, $\theta_{\{t,k\}}$)
2:     Compute cost
3:     $c_{\{t+1,k+1\}} = \beta \cdot s_{\{t+1,k+1\}}[0,1]$
4:     Compute values from transition
5:     $V_w(s_{\{t+1,k+1\}}) = \mathbf{w} \cdot s_{\{t+1,k+1\}}$
6:     $V_w(s_{\{t,k\}}) = \mathbf{w} \cdot s_{\{t,k\}}$
7:     Compute TD error
8:     $d_{\{t,k\}} = c_{\{t+1,k+1\}} + \gamma \cdot V_w(s_{\{t+1,k\}}) - V_w(s_{\{t,k\}})$
9:     Update Critic's parameter
10:     $\mathbf{w}_{\{t+1,k+1\}} = \mathbf{w}_{\{t,k\}} + lr_c \cdot d_{\{t,k\}} \cdot z_{\{t,k\}}$
11:     Update the eligibility vector
12:     $z_{\{t+1,k+1\}} = \lambda \cdot z_{\{t,k\}} + s_{\{t+1,k\}}$
13:     Compute Actor's gradient
14:     $g_{\{t,k\}} = \dfrac{d_{\{t,k\}}}{s_{\{t,k\}}}$
15:     Update Actor's policy parameters
16:     $\theta_{\{t,k+1\}} = \text{Adam}(\theta_{\{t,k\}}, g_{\{t,k\}}, lr_a)$
17:     **return** $\theta_{\{t,k+1\}}$
18: **end function**
19:
20: **function Predict**(*agent*)
21:     Compute *LP* based on Equation (15)
22:     **if** *agent = Agent_PS* **then**
23:         Compute policy prediction $P_t$
24:         $P_t = LP_t$
25:         Compute corresponding $a_t$
26:         $a_t = a_{t,k-1} + m \cdot P_t \cdot a_{t,k-1}$
27:         **return** $a_t$
28:     **end if**
29:     **if** *agent = Agent_ICR* **then**
30:         Compute *SP* based on Equation (16)
31:         Compute policy prediction $P_t$
32:         $P_t = \alpha_{LP} \cdot LP_t + (1 - \alpha_{LP}) \cdot SP_t$
33:         Compute corresponding $a_t$
34:         $a_t = a_{t,k-1} + m \cdot P_t \cdot a_{t,k-1}$
35:         **return** $a_t$
36:     **end if**
37:     **if** *agent = Agent_Basal* **then**
38:         Compute policy prediction $P_k$
39:         $P_k = LP_k$
40:         Compute corresponding $a_k$
41:         $a_k = a_{k-1} + m \cdot P_k \cdot a_{k-1}$
42:         **if** $a_k < 0.25 \cdot TDD_{k-1}$ **then**
43:             $a_k = a_{k-1}$
44:         **end if**
45:         **return** $a_k$
46:     **end if**
47: **end function**

FIG. 8

FIG. 9

**Algorithm 1 ABBA - Pipeline**

1: <u>Data Collection</u>
2: Collect 2 weeks CGM/insulin under Equation (1)
3: <u>Initialisation</u>
4: **for** agent $\in \{Agent\_PS, Agent\_ICR, Agent\_Basal\}$ **do**
5:     Initialise actor policy parameters $\theta$ using TE
6:     Randomly initialise critic parameter vector **w**
7:     Filter CGM data and initialise hyper-parameters
8:     Initialise $a$ based on Section 3.3
9: **end for**
10: <u>On-Line learning</u>
11: **for** day $= k \in \mathscr{D}$ **do**
12:     Predict Basal insulin $Basl$
13:     $Basl = \text{Predict}(s_k^{bas}, \theta_k^{bas}, Agent\_Basal)$
14:     **for** meal step $= t \in \mathscr{M}$ **do**
15:         $PS = \text{Predict}(s_t^{PS}, \theta_t^{PS}, Agent\_PS)$
16:         $ICR = \text{Predict}(s_t^{ICR}, \theta_t^{ICR}, Agent\_ICR)$
17:         Compute Bolus insulin from Equation (3)
18:         Observe $s_{t+1}^{PS}, s_{t+1}^{ICR}$, and $CHO_{t+1}$
19:         $\theta_t^{PS} = \text{Update}(s_{t+1}^{PS}, s_t^{PS}, \theta_t^{PS})$
20:         $\theta_t^{ICR} = \text{Update}(s_{t+1}^{ICR}, s_t^{ICR}, \theta_t^{ICR})$
21:     **end for**
22:     Observe $s_{k+1}^{bas}$
23:     $\theta_{k+1}^{bas} = \text{Update}(s_{k+1}^{bas}, s_k^{bas}, \theta_k^{bas})$
24: **end for** end of simulation/real-world application

FIG. 10

**Europäisches Patentamt**
**European Patent Office**
**Office européen des brevets**

# EUROPEAN SEARCH REPORT

Application Number

EP 24 20 6753

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/043588 A1 (MOUGIAKAKOU STAVROULA [CH] ET AL) 6 February 2020 (2020-02-06) <br> * abstract; table 1 * <br> * paragraph [0001] - paragraph [0070] * <br> * paragraph [0215] - paragraph [0276] * <br> * paragraph [0337] - paragraph [0357] * <br> ----- | 1-15 | INV. <br> G16H20/17 <br> G16H50/20 |
| X | PANAGIOTOU MARIA ET AL: "A Complete AI-Based System for Dietary Assessment and Personalized Insulin Adjustment in Type 1 Diabetes Self-management", 20 September 2023 (2023-09-20), COMPUTER ANALYSIS OF IMAGES AND PATTERNS; [LECTURE NOTES IN COMPUTER SCIENCE; LECT.NOTES COMPUTER], SPRINGER NATURE SWITZERLAND, CHAM, PAGE(S) 77 - 86, XP047670406, ISSN: 0302-9743 ISBN: 978-3-031-44239-1 [retrieved on 2023-09-20] <br> * abstract; table 2 * <br> * page 80, paragraph 2 - page 83, paragraph 1 * <br> ----- | 1-15 | |
| X | US 2024/293618 A1 (JAFAR ADNAN [CA] ET AL) 5 September 2024 (2024-09-05) <br> * abstract * <br> * paragraph [0006] * <br> * paragraph [0027] - paragraph [0088] * <br> ----- | 1-15 | TECHNICAL FIELDS SEARCHED (IPC) <br><br> G16H |

The present search report has been drawn up for all claims

| Place of search | Date of completion of the search | Examiner |
|---|---|---|
| Munich | 13 March 2025 | Menschner, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 6753

13-03-2025

| Patent document cited in search report | Publication date | Patent family member(s) | | Publication date |
|---|---|---|---|---|
| US 2020043588 A1 | 06-02-2020 | AU | 2018235368 A1 | 10-10-2019 |
| | | AU | 2024200477 A1 | 15-02-2024 |
| | | CA | 3054108 A1 | 20-09-2018 |
| | | CN | 110678931 A | 10-01-2020 |
| | | EP | 3596732 A1 | 22-01-2020 |
| | | JP | 7261170 B2 | 19-04-2023 |
| | | JP | 2020513969 A | 21-05-2020 |
| | | RU | 2019130910 A | 19-04-2021 |
| | | US | 2020043588 A1 | 06-02-2020 |
| | | US | 2022254473 A1 | 11-08-2022 |
| | | WO | 2018167543 A1 | 20-09-2018 |
| | | WO | 2018167727 A1 | 20-09-2018 |
| US 2024293618 A1 | 05-09-2024 | US | 2024293618 A1 | 05-09-2024 |
| | | WO | 2024168257 A1 | 15-08-2024 |